Europäisches Patentamt

European Patent Office

Office européen des brevets

(19)

(11) Numéro de publication : **0 479 692 B1**

## FASCICULE DE BREVET EUROPEEN

(12)

(45) Date de publication du fascicule du brevet :
20.07.94 Bulletin 94/29

(51) Int. Cl.⁵ : **C07C 11/04,** C07C 5/48,
C07C 53/08, C07C 51/215

(21) Numéro de dépôt : **91420349.2**

(22) Date de dépôt : **03.10.91**

(54) **Procédé d'oxydation ménagée de l'éthane en un mélange d'éthylène et d'acide acétique.**

(30) Priorité : **05.10.90 FR 9012519**

(43) Date de publication de la demande :
**08.04.92 Bulletin 92/15**

(45) Mention de la délivrance du brevet :
**20.07.94 Bulletin 94/29**

(84) Etats contractants désignés :
**BE DE FR GB NL**

(56) Documents cités :
**EP-A- 0 261 264**
**EP-A- 0 407 091**
**FR-A- 1 333 494**
**US-A- 4 410 752**

(73) Titulaire : **RHONE-POULENC CHIMIE**
**25, quai Paul Doumer**
**F-92408 Courbevoie Cédex (FR)**

(72) Inventeur : **Barthe, Philippe**
**90, rue Sadi Carnot**
**F-93300 Aubervilliers (FR)**
Inventeur : **Blanchard, Gilbert**
**5, Allée des Acacias,**
**Lagny-le-Sec**
**F-60330 Le Plessis Belleville (FR)**

(74) Mandataire : **Dubruc, Philippe et al**
**RHONE-POULENC CHIMIE**
**Direction de la Propriété Industrielle**
**25, Quai Paul Doumer**
**F-92408 Courbevoie Cedex (FR)**

## Description

La présente invention concerne un procédé d'oxydation ménagée de l'éthane en un mélange d'éthylène et d'acide acétique.

L'acide acétique est un produit industriellement fabriqué dont les multiples usages sont largement connus. Les mélanges d'éthylène et d'acide acétique peuvent présenter en outre un intérêt particulier dès lors que, ce type de mélanges, selon sa composition précise, est susceptible de servir à alimenter un réacteur catalytique pour réaliser la synthèse de l'acétate de vinyle, monomère largement utilisé industriellement.

Dans la demande de brevet français n° 2 326 393 il a été proposé un procédé d'oxydéshydrogénation de l'éthane, le cas échéant, en présence d'eau pour produire de l'éthylène et de l'acide acétique. Le catalyseur utilisé dans cette réaction, exothermique et conduite en phase vapeur, est à base de molybdène et d'un ou plusieurs autres éléments.

Toutefois, les valeurs indiquées pour les rendements en acide acétique, avec ou sans addition d'eau à la charge gazeuse d'éthane, permettent de constater que l'acide acétique reste un produit minoritaire par rapport à l'éthylène.

Par ailleurs, des catalyseurs "témoins" à base de vanadium et d'antimoine, testés dans les conditions précitées conduisent soit à une activité de combustion de l'éthane soit à une transformation de l'éthane en éthylène.

Dans J. Catal. 52 (11) pages 116-132 (1978) est rappelé l'intérêt des catalyseurs à base de molybdène et de vanadium dans la réaction en cause et précisé que la formation de l'acide acétique dans ce procédé est fonction notamment de la pression totale.

Dans EP-A-0 261 264, concernant ce type de catalyseurs, il est indiqué que la pression partielle d'eau a une influence sur la formation de l'acide acétique.

Il est également connu d'après le brevet américain n° 4 410 752 de conduire la réaction d'oxydéshydrogénation de l'éthane en éthylène par mise en contact de l'éthane et d'un gaz contenant de l'oxygène en présence d'un catalyseur de formule $V P_a O_x$ dans laquelle a est compris entre 0,5 et 3,0 et x est une valeur choisie pour satisfaire les exigences de valences, ledit catalyseur pouvant renfermer le cas échéant un promoteur métallique. Un objectif central dans cette technique antérieure est de produire très sélectivement l'éthylène. Pour ce faire la réaction est conduite dans un réacteur en lit fixe à 300-600°C sous une pression voisine de la pression atmosphérique et avec un large excès (volumique) d'air par rapport à l'hydrocarbure. En outre il semble que le catalyseur du type phosphate de vanadyle utilisé dans cette technique antérieure doive présenter une structure cristallographique particulière. Les sélectivités en éthylène obtenues sont généralement supérieures à 50 %.

Il a maintenant été trouvé de manière tout à fait inattendue qu'il est possible d'obtenir principalement l'acide acétique par oxydation ménagée de l'éthane par l'oxygène à condition de mettre en oeuvre des catalyseurs particuliers qui seront plus amplement décrits dans ce qui suit et d'opérer sous une pression totale mesurée à 25°C supérieure ou égale à 2 bar relatifs. La présente invention a donc pour objet un procédé d'oxydation ménagée en phase vapeur de l'éthane par un gaz contenant de l'oxygène en un mélange d'éthylène et d'acide acétique caractérisé en ce que la réaction est conduite

a) en présence d'un catalyseur renfermant du vanadium, du phosphore et de l'oxygène déposés sur un support à base d'oxyde de titane et

b) sous une pression totale mesurée à 25°C supérieure ou égale à 2 bar relatifs.

Un aspect essentiel de la présente invention réside dans le catalyseur mis en oeuvre. Comme indiqué ci-avant ledit catalyseur renferme du vanadium, du phosphore et de l'oxygène déposés sur un support à base d'oxyde de titane.

L'oxyde de titane peut être choisi parmi les formes suivantes : $TiO_2$ anatase, $TiO_2$ rutile et $TiO_2(B)$. De préférence on recourt à $TiO_2$ anatase.

L'oxyde de titane représente généralement de 25 à 99,5 % en poids de la composition catalytique et, pour une bonne mise en oeuvre de l'invention, de 50 à 99 % poids.

La phase catalytique supportée par l'oxyde de titane et utilisée dans le cadre de la présente invention renferme par atome de vanadium au moins 0,1 atome de phosphore et de préférence, au moins 0,5 atome de phosphore, l'oxygène étant présent en la quantité requise pour satisfaire les exigences de valence de V et P dans la phase VPaOx. Pour des rapports atomiques P/V inférieurs à 0,5 une combustion plus ou moins importante est susceptible de survenir au détriment de la réaction désirée.

Il n'est pas utile que le phosphore soit présent dans des quantités dépassant 5,0 atomes de phosphore par atome de vanadium ; en effet, on observe dans ces conditions une diminution de la sélectivité en l'acide acétique. De préférence, la quantité de phosphore est inférieure ou égale à 3 atomes par atome de vanadium.

En général la quantité de vanadium présente dans la composition catalytique exprimée en % poids de

2

vanadium métalllique est comprise entre 0,05 et 35 %. Pour une bonne mise en oeuvre de l'invention elle représente de 0,20 à 10 % poids de ladite composition.

Les compositions catalytiques utiles à la mise en oeuvre du présent procédé peuvent être préparées de diverses manières. En effet la préparation de catalyseurs $VP_aO_x$ supportés est connue de l'art antérieur. On peut citer comme exemples les catalyseurs $VP_aO_x$ supportés sur silice et sur phosphate d'aluminium obtenus par imprégnation des supports correspondants par une solution aqueuse résultant de l'attaque de $NH_4VO_3$ par l'acide tartrique en milieu aqueux et addition d'acide orthophosphorique [J. Catal. 117 pages 301-310 (1989)] ou encore les catalyseurs $VP_aO_x$ supportés sur alumine obtenus par imprégnation du support correspondant par une solution aqueuse résultant de l'attaque de $V_2O_5$ par de l'acide oxalique en milieu aqueux et addition d'acide orthophosphorique [Appl. Catal. 45 pages 1-7 (1988)].

D'une manière générale, la préparation demande la mise en solution d'un composé du vanadium et l'addition en quantité voulue d'acide orthophosphorique avant imprégnation du support par la solution ainsi obtenue. Cette solution peut être obtenue à partir de la mise en solution d'un composé du vanadium pentavalent ($V_2O_5$, $VOCl_3$, $VOBr_3$, $VCl_5$, $NH_4VO_3$) ou bien à partir de la mise en solution d'un composé du vanadium tétravalent ($VO_2$, $VOSO_4$, $VOCl_2$, $VOBr_2$, $VCl_4$) ou bien par la réduction dans le milieu d'un composé du vanadium pentavalent ($V_2O_5$, $VOCl_3$, $VOBr_3$, $VCl_5$, $NH_4VO_3$) ; suivie de l'addition d'acide orthophosphorique en quantité voulue.

On peut utiliser comme réducteurs du composé du vanadium pentavalent les acides organiques tels l'acide oxalique, l'acide citrique, l'acide tartrique et l'acide maléique [cf. J. Catal. 34 pages 345-355 (1974)], l'hydrazine comme décrit dans le brevet américain n° 4 410 752 ou encore l'acide chlorhydrique concentré seul ou en présence d'acides à caractère non oxydant tels que l'acide formique, l'acide acétique, l'acide lactique et l'acide borique comme décrit dans le brevet américain n° 4 085 122.

Le support est ensuite imprégné par cette solution, séché dans l'air et calciné. La calcination est effectuée dans l'air entre 250 et 900°C et de préférence entre 350 et 750°C.

Ces compositions catalytiques peuvent se présenter sous diverses formes telles des poudres, des billes, des extrudés et des concassés.

Les compositions catalytiques préférées sont consituées par
- de 50 à 99 % en poids d'oxyde de titane anatase
- d'une phase de $V\ P_a\ O_x$ telle que le vanadium métallique représente de 0,20 à 10 % en poids et a est compris entre 0,5 et 3,0 (inclus) et où x a la valeur requise pour satisfaire les exigences de valences.

Les compositions catalytiques sont avantageusement mises en oeuvre dans le cadre du présent procédé en étant elles-mêmes déposées sur des particules de solide ou en enrobant des particules de solide à l'aide desdites compositions.

Lorsqu'un tel solide est utilisé il peut être choisi parmi les matériaux classiquement utilisés pour préparer des catalyseurs supportés, sous réserve, bien entendu, que ces matériaux soient chimiquement inertes vis-à-vis de la réaction. A titre d'exemples de tels solides, on peut citer : les silices, les alumines, les oxydes de magnésium, zirconium, lanthane, cérium, les argiles, les zéolithes, les carbures de silicium et les mélanges de ces composés.

Lorsque de tels solides sont mis en oeuvre pour préparer les catalyseurs utilisés dans la présente invention ils représentent de 20 à 92 % en poids de l'ensemble.

Conviennent plus particulièrement à cette préparation, des billes d'argile, la composition catalytique renfermant du vanadium, du phosphore et de l'oxygène supportés par de l'oxyde de titane, enrobant ces billes.

Comme indiqué en tête du présent mémoire, une autre caractéristique essentielle du procédé selon l'invention réside dans l'application d'une pression totale mesurée à 25°C supérieure ou égale à 2 bar relatifs. Il n'est pas utile que cette pression dépasse 100 bar relatifs. Pour une bonne mise en oeuvre de l'invention, la pression totale mesurée à 25°C devra être comprise entre 2 et 30 bar relatifs.

Selon la présente invention l'éthane est mis en contact avec un gaz contenant de l'oxygène. Compte-tenu du caractère fortement exothermique des réactions mises en jeu et des contraintes apportées par le risque d'auto-inflammation des mélanges comportant de l'éthane et de l'oxygène sous pression, il est préférable d'utiliser un mélange réactionnel comportant un excès d'alcane par rapport à l'oxygène pour conserver à la réaction son caractère catalytique et sélectif. Pour une bonne mise en oeuvre de la présente invention, le rapport volumique éthane/oxygène est supérieur à 1. Il est de préférence compris entre 6 et 60.

Dans le cadre de la présente invention on peut utiliser de l'air, de l'air appauvri par un gaz inerte, de l'air enrichi par de l'oxygène ou bien de l'oxygène pur. De préférence, on utilise de l'oxygène pur.

De l'eau peut également être additionnée au mélange réactionnel. Pour une bonne mise en oeuvre de l'invention le rapport volumique eau/éthane est compris entre 0 et 1,5. Il est de préférence compris entre 0,02 et 0,7.

La réaction de l'éthane et du gaz contenant de l'oxygène en présence de catalyseur défini précédemment est effectuée en phase vapeur. La température de réaction est en général comprise entre 150 et 600°C. La limite supérieure est fixée par la température d'auto-inflammation du mélange réactionnel qui est une fonction de la pression totale et du temps de séjour dans le réacteur du mélange réactionnel. Pour une bonne mise en oeuvre de la présente invention la température de réaction est comprise entre 175 et 400°C.

Le temps de contact dans le réacteur (exprimé en secondes) défini comme le rapport du volume de catalyseur dans le réacteur (exprimé en $cm^3$) au débit total dans le réacteur (exprimé en $Ncm^3/s$) est compris entre 0,05 et 10 s. Il est de préférence compris entre 0,1 et 5 s.

L'acide acétique gazeux en sortie de réacteur peut par exemple être condensé et récupéré sous la forme d'une solution aqueuse. Il est ensuite extrait de cette solution aqueuse suivant des méthodes bien connues telles que la distillation, l'extraction par solvant ou la distillation extractive.

Les exemples ci-après illustrent l'invention.

Les résultats y sont exprimés en termes de taux de conversion de l'éthane, sélectivités en éthylène et acide acétique, productivité en acide acétique, ces grandeurs étant définies comme suit :

- Taux de conversion de l'éthane en % ($TT_{C_2H_6}$)

$TT_{C_2H_6}$ = (nb moles $C_2H_6$ entrée - nb moles $C_2H_6$ sortie) x 100/(nb moles $C_2H_6$ entrée )

- Sélectivité en éthylène en % en moles

$S_{C_2H_4}$ = (nb moles $C_2H_4$ sortie) x 100/(nb moles $C_2H_6$ entrée - nb moles $C_2H_6$ sortie)

- Sélectivité en acide acétique en % en moles

$S_{AcOH}$ = (nb moles $CH_3COOH$ sortie) x 100/(nb moles $C_2H_6$ entrée - nb moles $C_2H_6$ sortie)

La productivité en acide acétique $P_{AcOH}$ (en g/h/l catalyseur) est la masse d'acide acétique en grammes produite par heure et par litre de charge catalytique.

Mode opératoire des tests catalytiques :

20 $cm^3$ du catalyseur à tester sont placés dans un réacteur en inconel de 25 cm de longueur et de 2 cm de diamètre intérieur. L'activité catalytique est mesurée après 45 min sous flux réactionnel dans les conditions voulues de température et de pression. Les débits de gaz sont assurés par l'intermédiaires de débit-mètres massiques. Le débit d'eau est assuré par l'intermédiaire d'une pompe doseuse. Le liquide est vaporisé dans un réacteur en acier inoxydable dont la température est maintenue à 275°C. Celui-ci est rempli de 50 $cm^3$ de biles d'alumines de faible surface spécifique, préalablement calcinées à 850°C dans l'air pour assurer également le rôle de mélangeur. Le réacteur est porté en température par l'intermédiaire d'un bain de sable fluidisé.

Les performances sont calculées à partir des résultats fournis par deux analyseurs chromatographiques en phase gazeuse en ligne avec la sortie du réacteur. L'un muni d'un détecteur à conductibilité thermique permet de séparer et de doser dans leur ordre d'élution $CO_2$, $C_2H_4$, $C_2H_6$, $O_2$, $N_2$ et CO. L'autre, muni d'un détecteur à ionisation de flamme permet de séparer et de doser dans leur ordre d'élution $C_2H_4$, $C_2H_6$ et l'acide acétique.

Préparation et conditionnement des catalyseurs :

Exemple 1

Préparation d'un catalyseur A à base de $(VO)_2P_2O_7$ selon l'art antérieur :

240 ml d'alcool isobutylique et 160 ml d'alcool benzylique sont placés dans un réacteur en verre muni d'un agitateur rotatif type ancre, d'une ampoule de coulée, d'une indication de température, d'un réfrigérant et équipé d'un dispositif de chauffage.

40 g de $V_2O_5$ en poudre sont ajoutés à la solution précédente et la suspension résultante est portée à reflux à 110°C pendant 2 heures.

On ajoute ensuite 58,3 g d'acide orthophosphorique à 85 % par l'intermédiaire de l'ampoule de coulée.

La suspension est de nouveau portée à reflux à 110°C pendant 2 heures. Le solvant est ensuite éliminé par distilation.

La pâte obtenue est séchée à l'air à 150°C durant 6 heures. Le solide obtenu est broyé puis calciné dans l'air durant 15 heures à 400°C.

La poudre obtenue a été ensuite enrobée sur un support constitué de billes d'argile d'un diamètre de 5 mm.

La teneur en $(VO)_2P_2O_7$ après enrobage est de 16 % en poids.

Exemple 2

Préparation d'un catalyseur B à base de $Mo_{0,73} V_{0,18} Nb_{0,09}$ selon l'art antérieur :

4

On introduit dans un réacteur en verre muni d'un agitateur rotatif type ancre, d'une indication de température, d'un réfrigérant et équipé d'un dispositif de chauffage, 500 ml d'eau permutée, 57,6 g d'heptamolybdate d'ammonium en poudre $(NH_4)_6Mo_7O_{24}$, $4H_2O$ et 9,2 g de métavanadate d'ammonium en poudre $NH_4VO_3$.

La suspension est portée à reflux à 90°C sous agitation durant 30 min.

A la solution obtenue on rajoute après refroidissement 12,2 g d'éthoxyde de niobium $Nb(C_2H_5O)_5$.

La suspension obtenue est portée à reflux à 90°C sous agitation durant 2 heures. La suspension est alors concentrée à sec.

La pâte obtenue est séchée à l'air 12 heures à 120°C puis calcinée dans l'air durant 5 heures à 350°C.

La poudre obtenue a été ensuite enrobée sur le même support constitué de billes d'argile que dans le cas du catalyseur A.

La teneur en $Mo_{0,73}$ $V_{0,18}$ $Nb_{0,09}$ après enrobage est de 19 % en poids.

Exemple 3

Préparation d'un catalyseur C selon l'invention :

7,37 g d'acide oxalique en poudre sont mis en solution à chaud et sous agitation dans 16,25 ml d'eau permutée. Une fois l'acide oxalique dissout, on ajoute à cette solution 3,27 g de $V_2O_5$ en poudre.

Après dissolution complète du $V_2O_5$ à chaud et sous agitation, on laisse refroidir la solution.

A température ambiante, on rajoute à cette solution 2,83 ml d'acide orthophosphorique à 85 %. On obtient ainsi une solution qui constitue la solution d'imprégnation du support $TiO_2$.

Le support $TiO_2$ est du titane anatase en poudre présentant une aire spécifique de 105 $m^2$/g et un volume poreux total de 0,35 $cm^3$/g. 45 g de ce support sont imprégnés par la solution précédente dans un drageoir tournant.

La poudre résultante est séchée dans l'air 6 heures à 150°C puis calcinée dans l'air 3 heures à 500°C. Le solide récupéré après calcination est broyé. On conserve la fraction inférieure à 125 $\mu$m.

25 g de cette poudre sont alors enrobés sur 100 g de billes d'argile, de la même façon que pour les catalyseurs A et B.

Le catalyseur enrobé est de nouveau séché dans l'air 6 heures à 150°C puis calciné dans l'air 3 heures à 500°C. La teneur en catalyseur C après enrobage est de 12,7 % en poids.

Les conditions de test catalytique :

Les catalyseurs A, B et C ont été testés dans les conditions suivantes :

La charge catalytique est de 20 $cm^3$ de catalyseur enrobé sur les billes d'argile. Le débit d'éthane est de 60 Nl/h, le débit d'oxygène est de 4 Nl/h, le débit d'eau de 5 ml/h (exprimé en liquide) soit un rapport éthane/oxygène de 15 (en volume).

La pression totate mesurée à 25°C est de 10 bar relatifs.

Le temps de contact est de 1,03 s soit une VVH de 3510 $h^{-1}$.

Les résultats sont rassemblés dans le tableau I.

## TABLEAU I

| Catalyseur | Temp. °C | $TT_{C2H6}$ % | $S_{C2H4}$ % moles | $S_{AcOH}$ % moles | $P_{AcOH}$ g/h/l cata |
|---|---|---|---|---|---|
| A Selon l'art antérieur | 250 300 350 375 | 0 0,10 0,17 0,31 | 0 84 81 80 | 0 0 0 0 | 0 0 0 0 |
| B Selon l'art antérieur | 250 300 350 375 | 0 0,30 0,60 0,97 | 0 55 61 67 | 0 19 24 20 | 0 5 12 16 |
| C Selon l'invention | 250 300 350 | 0,35 1,22 2,76 | 12 16 23 | 82 65 43 | 23 64 95 |

Exemple 4 :

Préparation d'un catalyseur D selon l'invention :

3,15 g d'acide tartrique sont mis en solution à chaud et sous agitation dans 17 ml d'eau permutée.

Après dissolution, on ajoute à la solution 1,40 g de $V_2O_5$ en poudre.

Après dissolution à chaud et sous agitation de $V_2O_5$, on laisse la solution revenir à la température ambiante.

On ajoute alors à cette dernière 2,03 g d'acide orthophosphorique à 85 %. On obtient ainsi la solution d'imprégnation.

45 g de $TiO_2$ anatase (aire spécifique 45 m²/g, volume poreux total 0,38 cm³/g) sont imprégnés en drageoir tournant par la solution précédemment obtenue.

La poudre est séchée à l'air durant 6 heures à 150°C puis calcinée 3 heures à 500°C dans l'air.

25 g de la poudre obtenue sont enrobés sur 100 g de billes d'argile de diamètre 5 mm. Après séchage dans l'air à 150°C durant 6 heures et calcination dans l'air durant 3 heures à 500°C, le taux d'enrobage est de 17,3 %.

Exemple 5 :

Préparation d'un catalyseur E selon l'invention :

1,82 g d'acide citrique sont mis en solution à chaud et sous agitation dans 16 ml d'eau permutée.

Après dissolution, on ajoute à la solution 0,81 g de $V_2O_5$ en poudre.

Après dissolution à chaud et sous agitation de $V_2O_5$, on laisse la solution revenir à la température ambiante.

On ajoute alors à cette dernière 1,17 g d'acide orthophosphorique à 85 %. On obtient ainsi la solution d'imprégnation.

26 g de $TiO_2$ rutile (aire spécifique 30 m²/g, volume poreux total 0,58 cm³/g) sont imprégnés en drageoir tournant par la solution précédemment obtenue.

La poudre est séchée à l'air durant 6 heures à 150°C puis calcinée 3 heures à 400°C dans l'air.

25 g de la poudre obtenue sont enrobés sur 100 g de billes d'argile de diamètre 5 mm. Après séchage dans l'air à 150°C durant 6 heures et calcination dans l'air durant 3 heures à 400°C, le taux d'enrobage est de 16,6 %.

6

Exemple 6 :

Préparation d'un catalyseur F selon l'invention :

15 g d'acide oxalique sont mis en solution à chaud et sous agitation dans 25 ml d'eau permutée.

Après dissolution, on ajoute à la solution 6,04 g de $V_2O_5$ en poudre.

Après dissolution à chaud et sous agitation de $V_2O_5$, on laisse la solution revenir à la température ambiante.

On ajoute alors à cette dernière 22,85 g d'acide orthophosphorique à 85 %. On obtient ainsi la solution d'imprégnation.

45 g de $TiO_2$ anatase (aire spécifique 275 $m^2$/g, volume poreux total 1,18 $cm^3$/g) sont imprégnés en drageoir tournant par la solution précédemment obtenue.

La poudre est séchée à l'air durant 6 heures à 150°C puis calcinée 3 heures à 600°C dans l'air.

25 g de la poudre obtenue sont enrobés sur 100 g de billes d'argile de diamètre 5 mm. Après séchage dans l'air à 150°C durant 6 heures et calcination dans l'air durant 3 heures à 600°C, le taux d'enrobage est de 13,6 %.

Exemple 7 :

Préparation d'un catalyseur G selon l'invention :

8,2 g de sulfate de vanadyle $VOSO_4$, $5H_2O$ sont mis en solution à chaud et sous agitation dans 17 ml d'eau permutée.

Après dissolution, on laisse la solution revenir à la température ambiante.

On ajoute alors à cette dernière 1,84 g d'acide orthophosphorique à 85 %. On obtient ainsi la solution d'imprégnation.

45 g de $TiO_2$ anatase (aire spécifique 105 $m^2$/g, volume poreux total 0,35 $cm^3$/g) sont imprégnés en drageoir tournant par la solution précédemment obtenue.

La poudre est séchée à l'air durant 6 heures à 150°C puis calcinée 3 heures à 500°C dans l'air.

25 g de la poudre obtenue sont enrobés sur 100 g de billes d'argile de diamètre 5 mm. Après séchage dans l'air à 150°C durant 6 heures et calcination dans l'air durant 3 heures à 500°C, le taux d'enrobage est de 18,6 %.

Exemple 8 :

Préparation d'un catalyseur H selon l'invention :

1,33 g d'acide oxalique sont mis en solution à chaud et sous agitation dans 15 ml d'eau permutée.

Après dissolution, on ajoute à la solution 0,59 g de $V_2O_5$ en poudre. Après dissolution à chaud et sous agitation de $V_2O_5$, on laisse la solution revenir à la température ambiante.

On ajoute alors à cette dernière 0,86 g d'acide orthophosphorique à 85 %. On obtient ainsi la solution d'imprégnation.

19 g de $TiO_2$ (B) (aire spécifique 35 $m^2$/g, volume poreux 0,8 $cm^3$/g) sont imprégnés en drageoir tournant par la solution précédemment obtenue.

La poudre est séchée à l'air durant 6 heures à 150°C puis calcinée 3 heures à 350°C dans l'air.

20 g de la poudre obtenue sont enrobés sur 100 g de billes d'argile de diamètre 5 mm. Après séchage dans l'air à 150°C durant 6 heures et calcination dans l'air durant 3 heures à 600°C, le taux d'enrobage est de 14 %.

Les conditions de test catalytique :

Les catalyseurs D à H ont été testés dans les mêmes conditions que les catalyseurs A à C pour lesquels les conditions ont été précédemment décrites. Les résultats sont rassemblés dans le tableau II.

7

## TABLEAU II

| Catalyseur | Temp. °C | $TT_{C2H6}$ % | $S_{C2H4}$ % moles | $S_{AcOH}$ % moles | $P_{AcOH}$ g/h/l cata |
|---|---|---|---|---|---|
| D | 250 | 0,73 | 10,5 | 73,7 | 43 |
|   | 285 | 1,40 | 11,8 | 59,7 | 67 |
|   | 325 | 3,47 | 19,0 | 56,2 | 156 |
| E | 250 | 0,26 | 12,7 | 76,7 | 16 |
|   | 285 | 0,78 | 14,2 | 68,1 | 43 |
|   | 325 | 2,02 | 20,0 | 51,2 | 83 |
|   | 375 | 3,21 | 29,6 | 35,3 | 91 |
| F | 250 | 0,26 | 33,8 | 53,6 | 11 |
|   | 300 | 0,97 | 31,5 | 29,5 | 23 |
|   | 350 | 2,19 | 31,5 | 21,2 | 37 |
| G | 250 | 1,55 | 12,0 | 44,3 | 55 |
|   | 325 | 2,70 | 16,2 | 25,5 | 55 |
| H | 250 | 0,40 | 11,0 | 44,9 | 14 |
|   | 285 | 0,76 | 14,0 | 46,9 | 29 |
|   | 325 | 2,00 | 25,3 | 30,3 | 49 |
|   | 375 | 2,77 | 29,0 | 22,0 | 49 |

## Revendications

1. Procédé d'oxydation ménagée en phase vapeur de l'éthane par un gaz contenant de l'oxygène en un mélange d'éthylène et d'acide acétique caractérisé en ce que la réaction est conduite
   a) en présence d'un catalyseur renfermant du vanadium, du phosphore et de l'oxygène déposés sur un support à base d'oxyde de titane et
   b) sous une pression totale mesurée à 25°C supérieure ou égale à 2 bar relatifs.

2. Procédé selon la revendication 1, caractérisé en ce que l'oxyde de titane est choisi parmi $TiO_2$ anatase, $TiO_2$ rutile et $TiO_2$ (B).

3. Procédé selon la revendication 1 ou 2, caractérisé en ce que l'oxyde de titane est $TiO_2$ anatase.

4. Procédé selon l'une quelconque des revendications précédentes, caractérisé en ce que l'oxyde de titane représente de 25 à 99,5 % en poids de la composition catalytique ($VP_aO_x$, $TiO_2$) et, de préférence de 50 à 99 % en poids de ladite composition.

5. Procédé selon l'une quelconque des revendications précédentes, caractérisé en ce que le vanadium déposé représente de 0,05 à 35 % en poids de la composition catalytique ($VP_aO_x$, $TiO_2$).

6. Procédé selon l'une quelconque des revendications précédentes, caractérisé en ce que le vanadium déposé représente de 0,20 à 10 % en poids de la composition catalytique ($VP_aO_x$, $TiO_2$).

7. Procédé selon l'une quelconque des revendications précédentes, caractérisé en ce que dans la phase $VP_aO_x$ a est supérieur ou égal à 0,1 et, de préférence, supérieur ou égal à 0,5.

8. Procédé selon l'une quelconque des revendications précédentes, caractérisé en ce que dans la phase

VP$_a$O$_x$ a est inférieur ou égal à 5 et, de préférence, inférieur ou égal à 3.

9. Procédé selon l'une quelconque des revendications précédentes, caractérisé en ce que la composition catalytique (VP$_a$O$_x$, TiO$_2$) est déposée sur des particules de solide chimiquement inerte vis-à-vis de la réaction.

10. Procédé selon la revendication 9, caractérisé en ce que le solide représente de 20 à 92 % en poids de l'ensemble.

11. Procédé selon la revendication 9 ou 10, caractérisé en ce que les particules de solides sont des billes d'argiles et en ce que la composition catalytique enrobe lesdites billes.

12. Procédé selon l'une quelconque des revendications précédentes, caractérisé en ce que le rapport volumique éthane/oxygène est supérieur à 1.

13. Procédé selon la revendication 12, caractérisé en ce que le rapport volumique éthane/oxygène est compris entre 6 et 60.

14. Procédé selon l'une quelconque des revendications précédentes, caractérisé en ce que la température de réaction est comprise entre 150 et 600°C et, de préférence entre 175 et 400°C.


**Patentansprüche**

1. Verfahren zur schonenden Dampfphasenoxidation von Ethan mit einem Sauerstoff-enthaltenden Gas zu einem Gemisch aus Ethylen und Essigsäure, dadurch gekennzeichnet, daß die Reaktion
   a) in Gegenwart eines Katalysators, der Vanadium, Phosphor und Sauerstoff, abgeschieden auf einem Träger, auf der Basis von Titanoxid enthält, und
   b) unter einem Gesamtdruck von 2 bar relativ oder mehr, gemessen bei 25°C, durchgeführt wird.

2. Verfahren nach Anspruch 1, dadurch gekennzeichnet, daß das Titanoxid aus TiO$_2$ Anatas, TiO$_2$ Rutil und TiO$_2$ (B) ausgewählt wird.

3. Verfahren nach Anspruch 1 oder 2, dadurch gekennzeichnet, daß das Titanoxid TiO$_2$ Anatas ist.

4. Verfahren nach einem der vorangehenden Ansprüche, dadurch gekennzeichnet, daß das Titanoxid 25 bis 99,5 Gew.-% der katalytischen Zusammensetzung (VP$_a$O$_x$, TiO$_2$) und vorzugsweise 50 bis 99 Gew.-% der Zusammensetzung ausmacht.

5. Verfahren nach einem der vorangehenden Ansprüche, dadurch gekennzeichnet, daß das abgeschiedene Vanadium 0,05 bis 35 Gew.-% der katalytischen Zusammensetzung (VP$_a$O$_x$, TiO$_2$) ausmacht.

6. Verfahren nach einem der vorangehenden Ansprüche, dadurch gekennzeichnet, daß das abgeschiedene Vanadium 0,20 bis 10 Gew.-% der katalytischen Zusammensetzung (VP$_a$O$_x$, TiO$_2$) ausmacht.

7. Verfahren nach einem der vorangehenden Ansprüche, dadurch gekennzeichnet, daß in der Phase VP$_a$O$_x$ a den Wert 0,1 oder darüber und vorzugsweise den Wert 0,5 oder darüber hat.

8. Verfahren nach einem der vorangehenden Ansprüche, dadurch gekennzeichnet, daß in der Phase VP$_a$O$_x$ a 5 oder weniger bedeutet, vorzugsweise 3 oder weniger bedeutet.

9. Verfahren nach einem der vorangehenden Ansprüche, dadurch gekennzeichnet, daß die katalytische Zusammensetzung (VP$_a$O$_x$, TiO$_2$) auf Teilchen eines der Reaktion gegenüber inerten Feststoffs abgeschieden ist.

10. Verfahren nach Anspruch 9, dadurch gekennzeichnet, daß der Feststoff 20 bis 92 Gew.-% des gesamten Katalysators ausmacht.

11. Verfahren nach Anspruch 9 oder 10, dadurch gekennzeichnet, daß die Feststoffteilchen Tonkugeln sind und daß die katalytische Zusammensetzung diese Kugeln umhüllt.

12. Verfahren nach einem der vorangehenden Ansprüche, dadurch gekennzeichnet, daß das Volumenverhältnis Ethan/Sauerstoff über 1 liegt.

13. Verfahren nach Anspruch 12, dadurch gekennzeichnet, daß das Volumenverhältnis Ethan/Sauerstoff im Bereich von 6 bis 60 liegt.

14. Verfahren nach einem der vorangehenden Ansprüche, dadurch gekennzeichnet, daß die Reaktionstemperatur im Bereich von 150 bis 600°C und vorzugsweise im Bereich von 175 bis 400°C liegt.

## Claims

1. A process for the controlled oxidation in the vapour phase of ethane by a gas containing oxygen to give a mixture of ethylene and acetic acid characterised in that the reaction is carried out:
    a) in the presence of a catalyst containing vanadium, phosphorus and oxygen which are deposited on a support based on titanium oxide, and
    b) under a total pressure measured at 25°C which is higher than or equal to 2 bars relative.

2. A process according to claim 1 characterised in that the titanium oxide is selected from $TiO_2$ anatase, $TiO_2$ rutile and $TiO_2$ (B).

3. A process according to claim 1 or claim 2 characterised in that the titanium oxide is $TiO_2$ anatase.

4. A process according to any one of the preceding claims characterised in that the titanium oxide represents from 25 to 99.5% by weight of the catalytic composition ($VP_aO_x$, $TiO_2$) and preferably from 50 to 99% by weight of said composition.

5. A process according to any one of the preceding claims characterised in that the deposited vanadium represents from 0.05 to 35% by weight of the catalytic composition ($VP_aO_x$, $TiO_2$).

6. A process according to any one of the preceding claims characterised in that the deposited vanadium represents from 0.20 to 10% by weight of the catalytic composition ($VP_aO_x$, $TiO_2$).

7. A process according to any one of the preceding claims characterised in that in the phase $VP_aO_x$ a is greater than or equal to 0.1 and preferably greater than or equal to 0.5.

8. A process according to any one of the preceding claims characterised in that in the phase $VP_aO_x$ a is less than or equal to 5 and preferably less than or equal to 3.

9. A process according to any one of the preceding claims characterised in that the catalytic composition ($VP_aO_x$, $TiO_2$) is deposited on particles of solid which is chemically inert with respect to the reaction.

10. A process according to claim 9 characterised in that the solid represents from 20 to 92% by weight of the total.

11. A process according to claim 9 or claim 10 characterised in that the particles of solids are balls of clay and that the catalytic composition encases said balls.

12. A process according to any one of the preceding claims characterised in that the ethane/oxygen ratio by volume is higher than 1.

13. A process according to claim 12 characterised in that the ethane/oxygen ratio by volume is between 6 and 60.

14. A process according to any one of the preceding claims characterised in that the reaction temperature is between 150 and 600°C and preferably between 175 and 400°C.